# EUROPEAN PATENT SPECIFICATION

(11) **EP 1 054 862 B1**
(45) Date of publication and mention of the grant of the patent: **22.09.2004**
(21) Application number: 99932496.5
(22) Date of filing: 12.02.1999
(51) Int. Cl.: C07C 319/00

(54) **PROCESS FOR THE PRODUCTION OF ORGANIC DISULPHIDES**
VERFAHREN ZUR HERSTELLUNG VON ORGANISCHEN DISULFIDEN
PROCEDE DE PRODUCTION DE BISULFURES ORGANIQUES

(30) Priority: 13.02.1998 US 619164
(43) Date of publication of application: 29.11.2000
(73) Proprietor: ALBEMARLE CORPORATION, Baton Rouge, LA 70801-1765 (US)
(72) Inventor: CHENG, Chi Hung, Baton Rouge, LA 70815 (US); SULZER, Gerald, M., Baton Rouge, LA 70808 (US)
(74) Representative: UEXKÜLL & STOLBERG
(86) International application number: PCT/US1999/002991
(87) International publication number: WO 1999/041234

(56) References cited:
- US-A- 5 659 086

## Description

### TECHNICAL FIELD

This invention relates to an improved process by which organic disulfides, especially lower alkyl disulfides, can be produced in high yield and with high efficiency.

### BACKGROUND

U.S. Pat. No. 5,659,086 describes a process for producing organic disulfides comprising the steps of (1) contacting a base and a mercaptan to form a brine phase comprising the base and a basic salt of a mercaptan; (2) contacting the brine phase with a mercaptan and hydrogen peroxide to form an aqueous phase and an organic phase; (3) separating the aqueous phase from the organic phase; (4) recovering the organic phase; and optionally, (5) removing a portion of water from the aqueous phase and thereafter repeating the steps of (2) to (5). The separation of step (3) is conducted by decantation, centrifugation, solvent extraction, chromatographic separation, or a combination of any two or more thereof, with decantation being preferred.

### SUMMARY OF THE INVENTION

This invention provides a novel, highly efficient process enabling production of organic disulfides in high yield and purity. Not only is the process very easy and simple to carry out, but in addition the process (i) eliminates the need for forming an organic phase of liquid organic disulfide product and conducting a separation between such product and the total aqueous phase used in the reaction, (ii) enables efficient control of a highly exothermic reaction, and (iii) minimizes alkanesulfonic acid salt formation during the reaction.

In accordance with one of the embodiments of this invention there is provided a process for production of organic disulfide, which process comprises producing in a reaction zone a vapor phase comprising organic disulfide from reaction between an aqueous solution of basic mercaptide salt and water-soluble peroxide, and concurrently recovering vapor phase from the reaction zone.

Contact between the peroxide and the aqueous solution of basic mercaptide salt results in an exothermic reaction taking place. By maintaining a suitably low pressure in the reaction zone, a vapor phase comprising organic disulfide product is formed and concurrently removed or withdrawn from the reaction zone. This vaporization consumes a portion of the heat of reaction produced. At the same time by operating under suitable reaction temperature and pressure conditions, a portion of the water can be caused concurrently to vaporize from the aqueous solution in the reaction zone thereby further consuming the exothermic heat of reaction produced. Consequently, the temperature in the reaction zone is more readily controlled and maintained. Thus in the preferred modes of operation a vapor phase is formed comprising the organic disulfide being produced and water resulting from vaporization of a portion of aqueous reaction medium, and this vapor phase is removed (withdrawn or recovered) from the reaction zone concurrently with its formation, e.g., substantially as soon as it is formed.

It will be seen, therefore, that in conducting the process it is desirable to feed or introduce at a uniform or substantially uniform rate one of the reactants (i.e., the peroxide or the aqueous solution of basic mercaptide salt) into the reaction zone containing the other such reactant. Such feed rate preferably results, under the reaction conditions being used, in formation of vapor phase at a rate corresponding or substantially corresponding to the rate at which the vapor phase is being withdrawn from the reaction zone, thus providing and maintaining steady state conditions in the reaction zone. The feed itself can be intermittent or continuous. Preferably, the peroxide is fed into the aqueous solution of basic mercaptide salt, and preferably such feed is continuous during at least a major portion (more than 50%) of the reaction period. Most preferably, the continuous feed of peroxide is maintained during substantially the entire reaction period. Another way of conducting this reaction is to feed both reactants into the reaction zone at rates that result in formation of vapor phase at a rate corresponding or substantially corresponding to the rate at which the vapor phase is being withdrawn from the reaction zone, thereby providing and maintaining steady state conditions in the reaction zone.

Thus this invention provides, *inter alia,* a process for production of organic disulfide, which process comprises reacting mercaptide and peroxide in an aqueous reaction medium under conditions such that organic disulfide is formed and is vaporized, and concurrently recovering vapor phase comprising at least vaporized organic disulfide formed in the reaction, and preferably a vapor phase further comprising water vaporized from the aqueous reaction medium.

In an embodiment of this process for production of organic disulfide, hydrogen peroxide is fed intermittently or continuously into an aqueous solution of alkali metal methyl mercaptide in the reaction zone and the vapor phase comprises dimethyl disulfide, wherein in said process optionally
a) the hydrogen peroxide used is approximately 70% aqueous hydrogen peroxide;
b) the alkali metal methyl mercaptide used is an aqueous solution of sodium methyl mercaptide;
c) the reaction is conducted at 55 to 65°C and 18.7 to 24.0 kPa (140 to 180 mm.Hg), absolute;
d) the hydrogen peroxide is fed at a constant or substantially constant flow rate;
e) the vapor phase withdrawn from the reaction zone is condensed to form a two-phase liquid mixture of a predominantly organic phase of dimethyl disulfide and an aqueous phase of predominantly water; and
f) the phases formed in e) are separated from each other.
The withdrawn vapor phase is condensed and can be dried and purified in any suitable manner, but preferably is distilled. As noted above, preferably the withdrawn vapor phase comprises both vaporized organic disulfide product and vaporized water, and in such case the vapors are condensed to form a two-phase mixture of a predominately organic phase of organic disulfide and an aqueous phase of predominately water; and most preferably, these phases are separated from each other by gravity separation. The separated organic phase can then be further purified to achieve whatever final product purity is desired. A portion of the separated aqueous phase can be, and preferably is, recycled in amounts sufficient to maintain material and energy balance.
In an preferred embodiment of the process according to the invention 50 to 70% aqueous hydrogen peroxide and an aqueous solution of sodium methyl mercaptide or potassium methyl mercaptide are mixed together in the reaction zone, and the vapor phase comprises dimethyl disulfide. Preferably the reaction is conducted at 55. to 65°C and one 18.7 to 24.0 kPa (140 to 180 mm Hg), absolute, and optionally the vapor phase recovered from the reaction zone is condensed to form a two-phase liquid mixture of a predominantly organic phase of dimethyl disulfide and an aqueous phase of predominantly water, said phases are separated from each other; and the separated organic phase is distilled so as to isolate dimethyl disulfide in dried, purified form.

In each of the above embodiments, it is particularly preferred to utilize reaction conditions in the reaction between peroxide and mercaptide that cause or result in vaporization of liquid water from the reaction medium at a rate sufficient to consume in the range of 50-100% of the exothermic heat of reaction resulting from the exothermic reaction that is occurring in that reaction zone. Also, additional heat above the heat of reaction can be put into the reactor, e.g., by means of a heating jacket, to effect even greater stripping of organic disulfide from the reactor. This minimizes the amount of dissolved disulfide in the aqueous phase which otherwise can react to form sulfonic acid with attendant losses of the desired product and of the inorganic base being used.

In conducting the process the amount of water present in the reaction mixture in b). should be at least sufficient to keep the reactants and reaction products in solution, but without use of an excessive amount of water above such amount. Thus it is desirable to proportion the feeds such that at the outset of the reaction between peroxide and mercaptide, the aqueous base solution contains about 15 wt% of the base such as sodium or potassium hydroxide, and to control the feeds such that the aqueous reaction solution contains no more than 35 wt% of dissolved salts, and preferably in the range of 20 to 30 wt% of dissolved salts. Thus water can be fed to the reaction mixture in b) periodically as needed to maintain the reaction mixture in solution, and at least a portion of the water can be introduced as a peroxide solution, preferably as a solution of hydrogen peroxide.

Usually, the peroxide is introduced to the reaction mixture in the form of a solution, and typically as an aqueous solution which generally is in the range of a 1 to 70% peroxide solution. In order to maintain a highly desirable material and energy balance in the reaction system of b), it is preferred to use a highly concentrated aqueous hydrogen peroxide solution, e.g., in the range of 50 to 70%, and to recycle a portion of the water recovered from the reaction mixture. This minimizes the amount of water that has to be disposed as plant effluent.

It is preferred to periodically discharge the entire reaction mass to purge out by-product sulfonic acid salt which will accumulate over time. Alternatively a continuous purge can be used to remove such by-product salt.

The above and other embodiments and features of this invention will be still further apparent from the ensuing description, the accompanying drawings, and the appended claims.

### BRIEF DESCRIPTION OF THE DRAWING

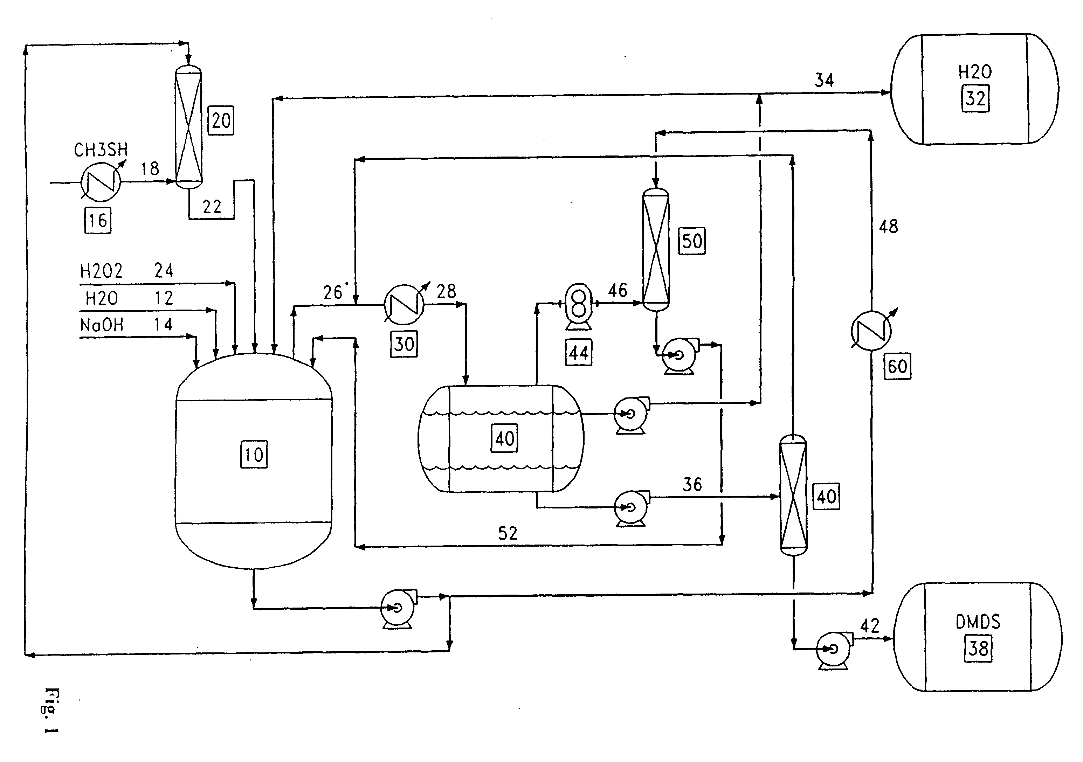

Fig. 1 is a schematic flow diagram of an installation for carrying out the process of this invention in a preferred manner on a semi-continuous basis.

### FURTHER DETAILED DESCRIPTION

For purposes of illustration, the operation of the installation depicted in Fig. 1 will be described with reference to production of dimethyl disulfide using sodium hydroxide and hydrogen peroxide as reagents in the process. It will be appreciated, however, that the same principles of operation can be utilized in the production of other suitably volatile organic disulfides. In the installation depicted, water and sodium hydroxide are fed to reactor **10** via lines **12** and **14,** respectively, to produce a solution in the range of 5 to 20 wt%, preferably 10 to 15 wt% NaOH. This solution is pumped to scrubber/neutralizer column **20,** which preferably is fabricated from stainless steel or other suitable corrosion resistant material. Methyl mercaptan or other suitable hydrocarbyl mercaptan is vaporized in heat exchanger **16** and continuously fed via line **18** into column **20**, where it reacts with the sodium hydroxide solution to form sodium mercaptide. Alternatively, the methyl mercaptan can be fed into column 20 in a liquid phase. Column 20 is operated at a pressure in the range of 0 to 68,95 kPa gauge to 10 psig and preferably in the range of 6,89 to 34,47kPa gauge (1 to 5 psig). The resulting aqueous reaction mixture containing sodium mercaptide and sodium hydroxide is transferred to reactor **10** via drain line **22**.

An aqueous solution of hydrogen peroxide, preferably as an approximately 70% solution, is fed, preferably at a constant rate, via line **24** into reactor **10** where the sodium mercaptide is oxidized to dimethyl disulfide. In this operation reactor **10** is maintained at a temperature in the range of 20 to 110°C and at a pressure in the range of (50 to 760 mm Hg) 6.7 to 101.3 kPa, and preferably at a temperature in the range of 50 to 75°C and at a pressure in the range of (140 to 180 mm Hg) 18.7 to 24.0 kPa. The exothermic reaction typically provides sufficient heat energy under the conditions employed to flash the dimethyl disulfide from reactor **10** substantially as rapidly as it is being formed. In addition, under the conditions employed, a portion of the water is also being flashed from the reactor, and this is of material assistance in controlling the exothermic reaction taking place in reactor **10**. The vapors exiting reactor 10 are transferred via line **26** to condenser **30** and the condensed liquids are transferred via line **28** to liquid/liquid separator **40** where the water and dimethyl disulfide phases are separated. The top phase is an aqueous phase typically substantially saturated with dimethyl disulfide (ca. 3000 ppm), and a portion is recycled back to reactor **10.** A purge stream of the remainder of the aqueous phase is transferred to storage tank **32** via line **34.** The dimethyl disulfide bottom phase, typically substantially saturated with water, is fed via line **36** to column **40** where the water content of the dimethyl disulfide is reduced from ca. 1300 ppm down to less than 50 ppm. During the distillation the dimethyl disulfide is not only dried but purified as well. The dry, purified dimethyl disulfide is sent to storage tank **38** via line **42**.

Noncondensables, including methyl mercaptan, exiting condenser **30** and after leaving vacuum pump **44** are sent via line **46** to scrubber column **50.** Column **50** is preferably fabricated from corrosion resistant material such as stainless steel. Scrubbing fluid for column **50** is pumped from reactor **10,** through exchanger **60** and line **48** to column **50,** and thereafter is returned to reactor **10** via line **52.**

### Mercaptide Formation

In the embodiments of this invention wherein the mercaptide is specifically formed for use in the process, the reaction can be illustrated using sodium hydroxide as the base, by the equation:

2 RSH + 2 NaOH - 2 RSNa + 2 H₂O

Thus typically the reactants are fed in a ratio of 0.8 to 2 equivalents of base per mole of mercaptan. To minimize loss of volatile mercaptan in the vapor phase during the ensuing recovery of the organic disulfide product, it is preferred to use enough base in the mercaptide-forming reaction to provide in the range of 0.1 to 10 wt% excess, and more preferably in the range of 0.1 to 5 wt% excess, of free base over and above that consumed in forming the mercaptide. The base is typically employed as an aqueous solution.

Mercaptans useful in the process of this invention are typically hydrocarbyl mercaptans such as alkyl, cycloalkyl, aryl, and aralkyl mercaptans, which usually contain no more than 10 carbon atoms. Preferred reactants are alkyl mercaptans having up to four carbon atoms, with methyl mercaptan and ethyl mercaptan being particularly preferred. The operating temperature and pressure should be adjusted such that the resulting disulfide is substantially vaporized in the reactor as it is formed. Thus if the mercaptan has a relatively high boiling point, a lower pressure or higher temperature, or both, should be used.

The base used in forming the aqueous basic or alkaline solutions used in forming the mercaptide is an inorganic base such as an alkali metal oxide, hydroxide, carbonate, bicarbonate, amide, or other basic salt, or an alkaline earth metal oxide, hydroxide, carbonate, bicarbonate, amide, or other basic salt. Examples include sodium oxide, sodium hydroxide, potassium oxide, potassium hydroxide, potassium carbonate, calcium oxide, calcium hydroxide, barium hydroxide, and like bases. Preferred are sodium hydroxide, potassium hydroxide, sodium oxide, and potassium oxide. In other words, aqueous solutions of sodium hydroxide and potassium hydroxide are preferred.

If the basicity of the aqueous solution used in the mercaptide forming step is relatively high and the aqueous solution is not diluted before conducting the peroxide oxidation, there is a possibility that some of the peroxide will be prematurely decomposed when performing the peroxide oxidation reaction. In such cases, the concentration of the aqueous base solution in the mercaptide-forming step when using a strong base such as sodium hydroxide or potassium hydroxide is typically kept in the range of 2 to 50 wt%, and preferably in the range of 5 to 15 wt%.

The mercaptide-forming step can be conducted under any suitable temperature and pressure conditions. Typically, temperatures in the mercaptide-forming reaction are in the range of 20 to 100°C. The pressure at which the reaction is performed is largely discretionary - the reaction can be performed at atmospheric pressure, at sub-atmospheric pressure or at super-atmospheric pressure.

### Organic Disulfide Formation

This reaction is illustrated by the following equation for the reaction between a sodium mercaptide and hydrogen peroxide:

2 RSNa + H₂O₂ - RSSR + 2 NaOH

In general, therefore, the reactants in this process are typically fed to the reactor in a ratio of 0.05 to 0.5 equivalents of peroxide per equivalent of mercaptide, including recycled mercaptide, if recycle is used. Overall about 0.5 mole of hydrogen peroxide is consumed per mole of the mercaptide.

In the mercaptide oxidation reaction, peroxide is typically used as the oxidant although other peroxy compounds such as hydroperoxides, persulfates, peroxycarbonates, superoxides, and like substances may be useable in lieu of peroxide or in addition thereto. Use of inorganic peroxides such as sodium peroxide, inorganic peroxy-acids (e.g., peroxystannic acid, peroxyvanadic acid, etc.), barium peroxide, zinc peroxide, thorium peroxide, and especially hydrogen peroxide, is preferable as such materials do not contribute organic residues to the reaction mixture and thus purification of the organic disulfide is facilitated. However it is within the scope of this invention to use an organic peroxide, if desired. When using hydrogen peroxide, it is preferably employed as a 10 to 70% or more aqueous solution, and 50 to 70% aqueous hydrogen peroxide solutions are most preferred.

While the mercaptide reaction mixture and the peroxide can be mixed together in any suitable manner, it is preferable to introduce the peroxide into the aqueous mercaptide reaction product mixture or into a freshly-prepared aqueous solution of the mercaptide at a controlled, extended rate of addition to prevent the virtually instantaneous, highly exothermic reaction from becoming excessively hot and uncontrollable. The controlled addition itself can be continuous or discontinuous, or a combination of both modes of addition of the peroxide. The reaction should be conducted at a reduced pressure sufficiently low, and the temperature should be controlled and maintained, such that the organic disulfide product being formed will vaporize from the reaction mixture essentially as soon as it is formed. Likewise, the conditions used preferably enable a portion of the water in the reaction mixture to vaporize along with the organic disulfide to thereby help maintain a well-controlled exothermic reaction under more-or-less steady state conditions. Typically the temperatures will be maintained within the range of 20 to 110°C, with pressures in the range of 6,7 to 101,3 kPa (50 to 760 mm Hg).

### Concurrent Reactions

In the embodiments of this invention wherein the mercaptide is specifically formed for use in the process, the mercaptide formation reaction and the mercaptide oxidation reaction to form the disulfide product in many cases can be performed concurrently in the same reaction vessel. This is accomplished by concurrently feeding all of the reaction components to the reactor and adjusting and maintaining the reaction conditions as described above such that the reactions identified at the outset as a) and b) occur concurrently. It is believed that as soon as the mercaptide is formed *in situ,* it is oxidized to the disulfide.

## Claims

1. A process for production of organic disulfide, which process comprises producing in a reaction zone a vapor phase comprising organic disulfide from reaction between an aqueous solution of basic mercaptide salt and water-soluble peroxide, and concurrently recovering vapor phase from the reaction zone.

2. A process according to claim 1 wherein said vapor phase also comprises vaporized water.

3. A process according to claim 2 wherein said basic mercaptide salt is an alkali metal mercaptide salt.

4. A process according to claim 2 wherein said basic mercaptide salt is sodium methyl mercaptide or sodium ethyl mercaptide.

5. A process according to any of claims 2 to 4 wherein said peroxide is hydrogen peroxide.

6. A process according to claim 1 further comprising forming the aqueous solution of basic mercaptide salt by reacting a mercaptan with a base in an aqueous medium.

7. A process according to claim 6 wherein said vapor phase also comprises vaporized water.

8. A process according to claim 7 wherein said base is an alkali metal base and said basic mercaptide salt is an alkali metal mercaptide salt.

9. A process according to claim 7 wherein said mercaptan is methyl mercaptan or ethyl mercaptan, wherein said

10. A process according to any of claims 7 to 9 wherein said peroxide is hydrogen peroxide.

11. A process according to claim 7 wherein liquid water is vaporized from the reaction medium of a rate that consumes at least 50% of the exothermic heat of reaction resulting from the reaction.

12. A process according to claim 11 wherein the aqueous solution of basic mercaptide salt is an aqueous solution of alkali metal methyl mercaptide or alkali metal ethyl mercaptide, and wherein the peroxide is hydrogen peroxide or an aqueous solution thereof.

13. A process according to claim 6 further comprising supplying heat energy to the reaction mixture over and above the exothermic heat of reaction resulting from said reaction.

14. A process according to claim 8 wherein the forming of the aqueous solution of basic mercaptide salt, and the producing in the reaction zone of said vapor phase are conducted concurrently in the same reactor.

15. A process according to claim 1 wherein the organic disulfide is formed by reaction of mercaptide and peroxide in an aqueous reaction medium and wherein organic disulfide is vaporized concurrently.

16. A process according to claim 15 wherein liquid water is vaporized from the reaction medium at a rate that consumes at least 50% fo the exothermic heat of reaction resulting from the reaction.

17. A process according to claim 16 wherein the mercaptide in the form fed to the reaction is a basic mercaptide salt or an aqueous solution thereof, and wherein the peroxide in the form fed to the reaction is hydrogen peroxide or an aqueous solution thereof.

18. A process according to claim 15 further comprising supplying heat energy to the reaction mixture over and above the exothermic heat of reaction resulting from said reaction.

19. A process according to claim 1 wherein hydrogen peroxide is fed intermittently or continuously into an aqueous solution of alkali metal methyl mercaptide in the reaction zone, and wherein the vapor phase comprises dimethyl disulfide.

20. A process according to claims 15 or 19 wherein said vapor phase also contains water vapor.

21. A process according to claim 19 wherein liquid water is vaporized from the reaction medium at a rate that consumes at least 50% of the exothermic heat of reaction resulting from the reaction.

22. A process according to claim 19 further comprising supplying heat energy to the reaction mixture over and above the exothermic heat of reaction resulting from said reaction.

23. A process according to claim 19 wherein:
a) the hydrogen peroxide used is approximately 70% aqueous hydrogen peroxide;
b) the alkali metal methyl mercaptide used is an aqueous solution of sodium methyl mercaptide;
c) the reaction is conducted at 55 to 65°C and 18.7 to 24.0 kPa (140 to 180 mm Hg), absolute;
d) the hydrogen peroxide is fed at a constant or substantially constant flow rate;
e) the vapor phase withdrawn from the reaction zone is condensed to form a two-phase liquid mixture of a predominantly organic phase of dimethyl disulfide and an aqueous phase of predominantly water; and
f) the phases formed in e) are separated from each other.

24. A process according to claim 23 wherein aqueous phase separated in f) is recycled to the reaction zone.

25. A process according to claim 23 wherein dimethyl disulfide of the organic phase separated in f) is concurrently dried and purified by subjecting said organic phase to distillation.

26. A process according to claim 19 wherein the aqueous solution of alkali metal methyl mercaptide is formed by reacting methyl mercaptan with alkali metal base dissolved in an aqueous medium.

27. A process according to any of claims 20, 21, 23, 24 or 25 wherein the aqueous solution of the alkali metal methyl mercaptide is formed by feeding vaporized methyl mercaptan into an aqueous solution of alkali metal base.

28. A process according to claim 1 wherein 50 to 70% aqueous hydrogen peroxide and an aqueous solution of sodium methyl mercaptide or potassium methyl mercaptide are mixed together in the reaction zone, and wherein the vapor phase comprises dimethyl disulfide.

29. A process according to claim 28 wherein the reaction is conducted at 55 to 65°C and one 18.7 to 24.0 kPa (140 to 180 mm Hg), absolute.

30. A process according to claim 28 wherein the vapor phase recovered from the reaction zone is condensed to form a two-phase liquid mixture of a predominantly organic phase of dimethyl disulfide and an aqueous phase of predominantly water, and wherein said phases are separated from each other.

31. A process according to claim 28 wherein the reaction is conducted at 55 to 65°C and 18.7 to 24.0 kPa (140 to 180 mm Hg), absolute, wherein the vapor phase recovered from the reaction zone is condensed to form a two-phase liquid mixture of a predominantly organic phase of dimethyl disulfide and an aqueous phase of predominantly water, wherein said phases are separated from each other; and wherein the separated organic phase is distilled so as to isolate dimethyl disulfide in dried, purified form.

## Patentansprüche

1. Verfahren zur Herstellung von organischen Disulfiden, bei dem das Verfahren die Herstellung einer Dampfphase, die ein organisches Disulfid aus einer Umsetzung einer wässrigen Lösung von basischem Mercaptidsalz mit wasserlöslichem Peroxid umfasst, in einer Reaktionszone und gleichzeitig die Gewinnung der Dampfphase aus der Reaktionszone umfasst.

2. Verfahren nach Anspruch 1, bei dem die Dampfphase auch verdampftes Wasser umfasst.

3. Verfahren nach Anspruch 2, bei dem das basische Mercaptidsalz ein Alkalimetallmercaptidsalz ist.

4. Verfahren nach Anspruch 2, bei dem das basische Mercaptidsalz Natriummethylmercaptid oder Natriumethylmercaptid ist.

5. Verfahren nach einem der Ansprüche 2 bis 4, bei dem das Peroxid Wasserstoffperoxid ist.

6. Verfahren nach Anspruch 1, das ferner die Bildung der wässrigen Lösung von basischem Mercaptidsalz durch Umsetzung eines Mercaptans mit einer Base in einem wässrigen Medium umfasst.

7. Verfahren nach Anspruch 6, bei dem die Dampfphase auch verdampftes Wasser umfasst.

8. Verfahren nach Anspruch 7, in dem die Base eine Alkalimetallbase ist und das basische Mercaptidsalz ein Alkalimetallmercaptidsalz ist. '

9. Verfahren nach Anspruch 7, bei dem das Mercaptan Methylmercaptan oder Ethylmercaptan ist, die Base eine basische anorganische Natriumverbindung ist und das basische Mercaptidsalz Natriummethylmercaptid oder Natriumethylmercaptid ist.

10. Verfahren nach einem der Ansprüche 7 bis 9, bei dem das Peroxid Wasserstoffperoxid ist.

11. Verfahren nach Anspruch 7, bei dem flüssiges Wasser aus dem Reaktionsmedium mit einer Rate verdampft wird, die mindestens 50 % der sich aus der Umsetzung ergebenden exothermen Reaktionswärme verbraucht.

12. Verfahren nach Anspruch 11, bei dem die wässrige Lösung von basischem Mercaptidsalz eine wässrige Lösung von Alkalimetallmethylmercaptid oder Alkalimetallethylmercaptid ist und das Peroxid Wasserstoffperoxid oder eine wässrige Lösung desselben ist.

13. Verfahren nach Anspruch 6, das ferner die Versorgung der Reaktionsmischung mit Wärmeenergie aus der sich aus der Umsetzung ergebenden exothermen Reaktionswärme und darüber hinaus umfasst.

14. Verfahren nach Anspruch 8, bei dem die Bildung der wässrigen Lösung von basischem Mercaptidsalz und die Herstellung der Dampfphase in der Reaktionszone gleichzeitig in demselben Reaktor durchgeführt werden.

15. Verfahren nach Anspruch 1, bei dem das organische Disulfid durch Umsetzung von Mercaptid und Peroxid in einem wässrigen Reaktionsmedium gebildet wird und das organische Disulfid gleichzeitig verdampft wird.

16. Verfahren nach Anspruch 15, bei dem flüssiges Wasser aus dem Reaktionsmedium mit einer Rate verdampft wird, die mindestens 50 % der sich aus der Umsetzung ergebenden exothermen Reaktionswärme verbraucht.

17. Verfahren nach Anspruch 16, bei dem das Mercaptid, in der der Umsetzung zugesetzten Form, ein basisches Mercaptidsalz oder eine wässrige Lösung desselben ist und das Peroxid, in der der Umsetzung zugesetzten Form, Wasserstoffperoxid oder eine wässrige Lösung desselben ist.

18. Verfahren nach Anspruch 15, das ferner die Versorgung der Reaktionsmischung mit Wärmeenergie aus der sich aus der Umsetzung ergebenden exothermen Reaktionswärme und darüber hinaus umfasst.

19. Verfahren nach Anspruch 1, bei dem Wasserstoffperoxid periodisch oder kontinuierlich in eine wässrige Lösung von Alkalimetallmethylmercaptid in der Reaktionszone zugeführt wird und die Dampfphase Dimethyldisulfid umfasst.

20. Verfahren nach den Ansprüchen 15 oder 19, bei dem die Dampfphase auch Wasserdampf enthält.

21. Verfahren nach Anspruch 19, bei dem flüssiges Wasser aus dem Reaktionsmedium mit einer Rate verdampft wird, die mindestens 50 % der sich aus der Umsetzung ergebenden exothermen Reaktionswärme umfasst.

22. Verfahren nach Anspruch 19, das ferner die Versorgung der Reaktionsmischung mit Wärmeenergie aus der sich aus der Umsetzung ergebenden exothermen Reaktionswärme und darüber hinaus umfasst.

23. Verfahren nach Anspruch 19, bei dem:
a) das verwendete Wasserstoffperoxid ungefähr 70 % wässriges Wasserstoffperoxid ist;
b) das verwendete Alkalimetallmethylmercaptid eine wässrige Lösung von Natriummethylmercaptid ist;
c) die Umsetzung bei 55 bis 65 °C und absoluten 18,7 bis 24,0 kPa (140 bis 180 mm Hg) durchgeführt wird;
d) das Wasserstoffperoxid bei einer konstanten oder im Wesentlichen konstanten Fließrate zugeführt wird,
e) die aus der Reaktionszone entzogene Dampfphase kondensiert wird, um eine zweiphasige flüssige Mischung aus einer überwiegend organischen Phase aus Dimethyldisulfid und einer wässrigen Phase aus überwiegend Wasser zu bilden, und
f) die in e) gebildeten Phasen voneinander getrennt werden.

24. Verfahren nach Anspruch 23, bei dem die in f) abgetrennte wässrige Phase zu der Reaktionszone zurückgeführt wird.

25. Verfahren nach Anspruch 23, bei dem Dimethyldisulfid aus der in f) abgetrennten organischen Phase gleichzeitig getrocknet und gereinigt wird, indem die organische Phase einer Destillation unterworfen wird.

26. Verfahren nach Anspruch 19, bei dem die wässrige Lösung von Alkalimetallmethylmercaptid durch Umsetzung von Methylmercaptan mit in einem wässrigen Medium gelöster Alkalimetallbase gebildet wird.

27. Verfahren nach einem der Ansprüche 20, 21, 23, 24 oder 25, bei dem die wässrige Lösung von Alkalimetallmethylmercaptid durch Zufügen von verdampftem Methylmercaptan in eine wässrige Lösung von Alkalimetallbase gebildet wird.

28. Verfahren nach Anspruch 1, bei dem 50 bis 70 % wässriges Wasserstoffperoxid und eine wässrige Lösung von Natriummethylmercaptid oder Kaliummethylmercaptid in der Reaktionszone miteinander gemischt werden und die Dampfphase Dimethyldisulfid umfasst.

29. Verfahren nach Anspruch 28, bei dem die Umsetzung bei 55 bis 65 °C und absoluten 18,7 bis 24,0 kPa (140 bis 180 mm Hg) durchgeführt wird.

30. Verfahren nach Anspruch 28, bei dem die Dampfphase, die aus der Reaktionszone gewonnen wird, kondensiert wird, um eine zweiphasige flüssige Mischung aus einer überwiegend organischen Phase von Dimethyldisulfid und einer wässrigen Phase aus überwiegend Wasser zu bilden und die Phasen voneinander getrennt werden.

31. Verfahren nach Anspruch 28, bei dem die Umsetzung bei 55 bis 65 °C und absoluten 18,7 bis 24,0 kPa (140 bis 180 mm Hg) durchgeführt wird, die Dampfphase, die aus der Reaktionszone gewonnen wird, kondensiert wird, um eine zweiphasige flüssige Mischung aus einer überwiegend organischen Phase von Dimethyldisulfid und einer wässrigen Phase aus überwiegend Wasser zu bilden, diese Phasen voneinander getrennt werden und die abgetrennte organische Phase destilliert wird, um so Dimethyldisulfid in getrockneter, gereinigter Form zu isolieren.

## Revendications

1. Procédé de production de disulfure organique, ce procédé comprenant une production dans une zone de réaction, d'une phase vapeur comprenant un disulfure organique, par réaction entre une solution aqueuse d'un sel basique type mercaptide et un peroxyde soluble dans l'eau, et une récupération simultanée de la phase vapeur à partir de la zone de réaction.

2. Procédé selon la revendication 1, dans lequel ladite phase vapeur comprend également de l'eau vaporisée.

3. Procédé selon la revendication 2, dans lequel ledit sel basique de type mercaptide est un sel de type mercaptide et de métal alcalin.

4. Procédé selon la revendication 2, dans lequel ledit sel basique de type mercaptide est le méthyl mercaptide de sodium ou l'éthyl mercaptide de sodium.

5. Procédé selon l'une quelconque des revendications 2 à 4, dans lequel ledit peroxyde est le peroxyde d'hydrogène.

6. Procédé selon la revendication 1, comprenant en outre la formation de la solution aqueuse de sel basique de type mercaptide par réaction d'un mercaptan avec une base dans un milieu aqueux.

7. Procédé selon la revendication 6, dans lequel ladite phase vapeur comprend également de l'eau vaporisée.

8. Procédé selon la revendication 7, dans lequel ladite base est une base de métal alcalin, et ledit sel basique de type mercaptide est un sel de type mercaptide de métal alcalin.

9. Procédé selon la revendication 7, dans lequel ledit mercaptan est le méthyl mercaptan ou l'éthyl mercaptan, dans lequel ladite base est un composé basique inorganique du sodium, et dans lequel ledit sel basique de type mercaptide, est le méthyl mercaptide de sodium ou l'éthyl mercaptide de sodium.

10. Procédé selon l'une quelconque des revendications 7 à 9, dans lequel ledit peroxyde est le peroxyde d'hydrogène.

11. Procédé selon la revendication 7, dans lequel de l'eau liquide est vaporisée à partir du milieu réactionnel à un débit qui consomme au moins 50 % de la chaleur de réaction exothermique résultant de la réaction.

12. Procédé selon la revendication 11, dans lequel la solution aqueuse de sel basique de type mercaptide, est une solution aqueuse de méthyl mercaptide de métal alcalin ou d'éthyl mercaptide de métal alcalin, et dans lequel le peroxyde est du peroxyde d'hydrogène ou une solution aqueuse de celui-ci.

13. Procédé selon la revendication 6, comprenant en outre l'apport d'énergie thermique au mélange réactionnel en supplément de la chaleur de réaction exothermique résultant de ladite réaction.

14. Procédé selon la revendication 8, dans lequel la formation de la solution aqueuse de sel basique de type mercaptide, et la production dans la zone de réaction de ladite phase vapeur, sont effectuées simultanément dans le réacteur.

15. Procédé selon la revendication 1, dans lequel le disulfure organique est formé par réaction d'un mercaptide et d'un peroxyde dans un milieu réactionnel aqueux, et dans lequel le disulfure organique est simultanément vaporisé.

16. Procédé selon la revendication 15, dans lequel de l'eau liquide est vaporisée à partir du milieu réactionnel selon un débit qui consomme au moins 50 % de la chaleur de réaction exothermique résultant de la réaction.

17. Procédé selon la revendication 16, dans lequel le mercaptide sous la forme soumise à la réaction, est un sel basique de type mercaptide ou une solution aqueuse de celui-ci, et dans lequel le peroxyde sous la forme soumise à la réaction, est du peroxyde d'hydrogène ou une solution aqueuse de celui-ci.

18. Procédé selon la revendication 15, comprenant en outre l'apport d'énergie thermique au mélange réactionnel en supplément de la chaleur de réaction exothermique résultant de ladite réaction.

19. Procédé selon la revendication 1, dans lequel on fournit du peroxyde d'hydrogène par intermittence ou de manière continue dans une solution aqueuse de méthyl mercaptide de métal alcalin dans la zone de réaction, et dans lequel la phase vapeur comprend du disulfure de diméthyle.

20. Procédé selon la revendication 15 ou 19, dans lequel ladite phase vapeur contient également de la vapeur d'eau.

21. Procédé selon la revendication 19, dans lequel de l'eau liquide est vaporisée à partir du milieu réactionnel à un débit qui consomme au moins 50 % de la chaleur de réaction exothermique résultant de la réaction.

22. Procédé selon la revendication 19, comprenant en outre l'apport d'énergie thermique au mélange réactionnel en supplément de la chaleur de réaction exothermique résultant de ladite réaction.

23. Procédé selon la revendication 19, dans lequel :
a) le peroxyde d'hydrogène employé est du peroxyde d'hydrogène aqueux à environ 70 % ;
b) le méthyl mercaptide de métal alcalin employé est une solution aqueuse de méthyl mercaptide de sodium ;
c) la réaction est effectuée à 55 jusqu'à 65 °C et sous une pression absolue de 18,7 à 24,0 kPa (de 140 à 180 mm Hg) ;
d) le peroxyde d'hydrogène est introduit à un débit constant ou à peu près constant ;
e) la phase vapeur évacuée de la zone de réaction est condensée pour former un mélange liquide biphasique comprenant une phase organique prédominante à base de disulfure de diméthyle et une phase aqueuse comprenant principalement de l'eau ; et
f) les phases formées dans l'étape e) sont séparées l'une de l'autre.

24. Procédé selon la revendication 23, dans lequel la phase aqueuse séparée dans l'étape f) est recyclée dans la zone de réaction.

25. Procédé selon la revendication 23, dans lequel le disulfure de diméthyle de la phase organique séparée dans l'étape f), est simultanément séché et purifié en soumettant ladite phase organique à une distillation.

26. Procédé selon la revendication 19, dans lequel la solution aqueuse de méthyl mercaptide de métal alcalin est formée en faisant réagir du méthyl mercaptan avec une base de métal alcalin dissoute dans un milieu aqueux.

27. Procédé selon l'une quelconque des revendications 20, 21, 23, 24 ou 25, dans lequel la solution aqueuse du méthyl mercaptide de métal alcalin est formée en introduisant du méthyl mercaptan vaporisé dans une solution aqueuse de base de métal alcalin.

28. Procédé selon la revendication 1, dans lequel on mélange ensemble dans la zone de réaction, du peroxyde d'hydrogène aqueux à 50 jusqu'à 70 % et une solution aqueuse de méthyl mercaptide de sodium ou de méthyl mercaptide de potassium, et dans lequel la phase vapeur comprend du disulfure de diméthyle.

29. Procédé selon la revendication 28, dans lequel la réaction est effectuée à 55 jusqu'à 65 °C et sous une pression absolue de 18,7 à 24,0 kPa (de 140 à 180 mm Hg).

30. Procédé selon la revendication 28, dans lequel la phase vapeur récupérée à partir de la zone de réaction est condensée pour former un mélange liquide biphasique d'une phase organique prédominante à base de disulfure de diméthyle et d'une phase aqueuse comprenant principalement de l'eau, et dans lequel lesdites phases sont séparées l'une de l'autre.

31. Procédé selon la revendication 28, dans lequel la réaction est effectuée à 55 jusqu'à 65 °C et sous une pression absolue de 18,7 à 24,0 kPa (de 140 à 180 mmHg), dans lequel la phase vapeur récupérée à partir de la zone de réaction est condensée pour former un mélange liquide biphasique d'une phase organique prédominante à base de disulfure de diméthyle et d'une phase aqueuse comprenant principalement de l'eau, dans lequel lesdites phases sont séparées l'une de l'autre ; et dans lequel la phase organique séparée est distillée de façon à isoler le disulfure de diméthyle sous forme séchée et purifiée.
